# EUROPÄISCHE PATENTSCHRIFT

(11) **EP 0 146 135 B1**
(45) Veröffentlichungstag und Bekanntmachung des Hinweises auf die Patenterteilung: **21.04.1993**
(21) Anmeldenummer: 84115602.9
(22) Anmeldetag: 17.12.1984
(51) Int. Cl.: C07D 498/10, G03C 1/72

(54) **Photochrome Substanzen**
Photochromic substances
Substances photochromiques

(30) Priorität: 16.12.1983 DE 3345625; 16.12.1983 DE 3345624
(43) Veröffentlichungstag der Anmeldung: 26.06.1985
(60) Teilanmeldung: 92105128.0
(73) Patentinhaber: Optische Werke G. Rodenstock, 80469 München (DE)
(72) Erfinder: Melzig, Manfred, Dr., D-8031 Wessling (DE); Martinuzzi, Giuseppe, D-8031 Eichenau (DE)
(74) Vertreter: Schiller, Walter, Dr.

(56) Entgegenhaltungen:
- DE-A- 1 927 849
- GB-A- 2 029 410
- US-A- 357 862
- US-A- 4 215 012

## Beschreibung

Die Erfindung bezieht sich auf photochrome Substanzen, d.h. auf Substanzen, deren Einfärbung sich entsprechend der Umgebungsbeleuchtung in Intensität und Farbeindruck ändert.

Photochrome Substanzen haben eine Vielzahl von Anwendungsmöglichkeiten, beispielsweise werden sie zur Herstellung von photochromen Sonnenschutz-Brillengläsern aus Kunststoff, Brillenfassungen etc. verwendet.

Photochrome Substanzen sind beispielsweise aus US-A-3 562 172, US-A-3 578 602, US-A-4 215 010 oder der DE-A 1 927 849, der DE-A-29 26 255 oder der GB-A-2 029 410 bekannt.

Die aus diesen Druckschriften bekannten photochromen Substanzen bzw. Verbindungen haben jedoch unter anderem den Nachteil, daß der photochrome Effekt stark temperaturabhängig ist: Mit steigenden Umgebungstemperaturen wird die Eindunkelung bei gleicher Beleuchtungsstärke geringer.

Der Erfindung liegt die Aufgabe zugrunde, photochrome Substanzen bzw. Verbindungen anzugeben, deren photochromer Effekt sich auch mit steigender Temperatur nur wenig ändert, d.h. die auch bei steigender Temperatur gute Eindunkelungseigenschaften aufweisen.

Diese Aufgabe wird erfindungsgemäß durch Verbindungen gelöst, die in an sich bekannter Weise erhalten werden durch die Kondensation
- eines substituierten 2-Alkylenindolins 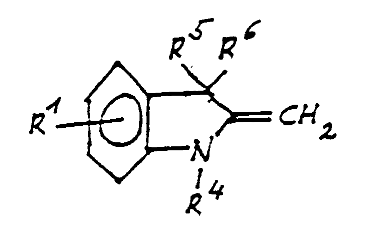
- mit einem 2-Hydroxy-1-nitroso-aromaten 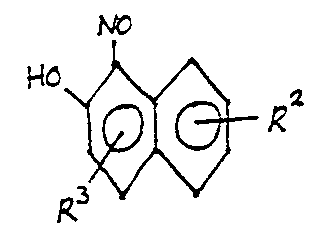
- in Anwesenheit eines Kondensationsmittels der Formel HNR₂, das den Rest -NR₂ in das Reaktionsprodukt einführt,
wobei die Reste
- R¹,R²,R³: einen oder mehrere Substituenten aus der Reihe -H, -CN, -SCN, NO₂, -X, -CH₂X, -CX₃, -OR, -SR, -COR, -COOR darstellen,
wobei X = Halogen und R = H, Alkyl mit bis zu 8 C-Atomen, Aryl, Heteroaryl sind bzw. annelierte aromatische oder heteroaromatische Ringsysteme bedeuten,
- R⁴,R⁵,R⁶: ein Substituent aus der Reihe -R⁷, -CH₂R⁷, ist,
wobei R⁷ = Alkyl mit bis zu 8 C-Atomen oder Aryl bedeutet, und
- R: Alkyl mit bis zu 8 C-Atomen, Aryl oder Cyclohexyl ist,
oder die beiden R zusammen mit dem N-Atom einen Piperidylrest bilden.

Die erfindungsgemäßen photochromen Verbindungen werden also durch Kondensation eines substituierten 2-Alkylenindolins mit einem 2-Hydroxy-1-nitroso-aromaten synthetisiert.

Die Bildung des 2-Alkylenindolins kann durch vorhergehende Synthese oder während des letzten Synthesenschritts aus einem entsprechenden Indoleninium Salz erfolgen. Die Einführung des Restes -NR₂ erfolgt in diesem Schritt über das Kondensationsmittel.

Anders ausgedrückt, wird eine Verbindung der allgemeinen - an sich bekannten - Formel 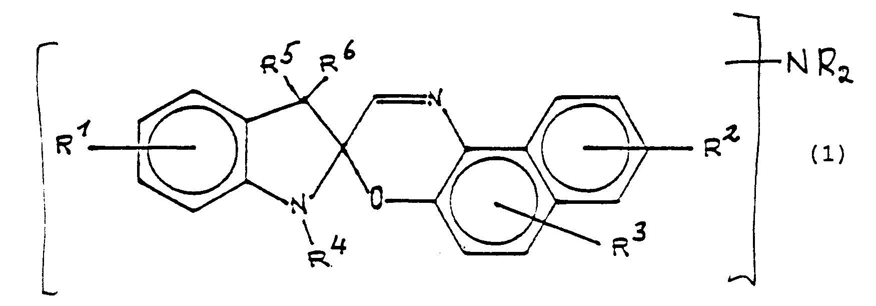
mit einem Substituenten -NR₂ an geeigneter, durch das erfindungsgemäße Herstellverfahren festgelegter Stelle versehen. Erfindungsgemäß ist nämlich erkannt worden, daß für die Verringerung der Temperaturabhängigkeit des photochromen Effekts die Reste R⁴, R⁵, R⁶ und -NR₂ entscheidend sind:
Durch die Einwirkung von kurzwelligem Licht öffnet sich die Spiro-C-O Bindung; nach der von den Erfindern entwickelten Modellvorstellung entstehen aus der Grundform, d.h. dem Molekül mit der allgemeinen Formel (1) folgende Moleküle, die miteinander im Gleichgewicht stehen: 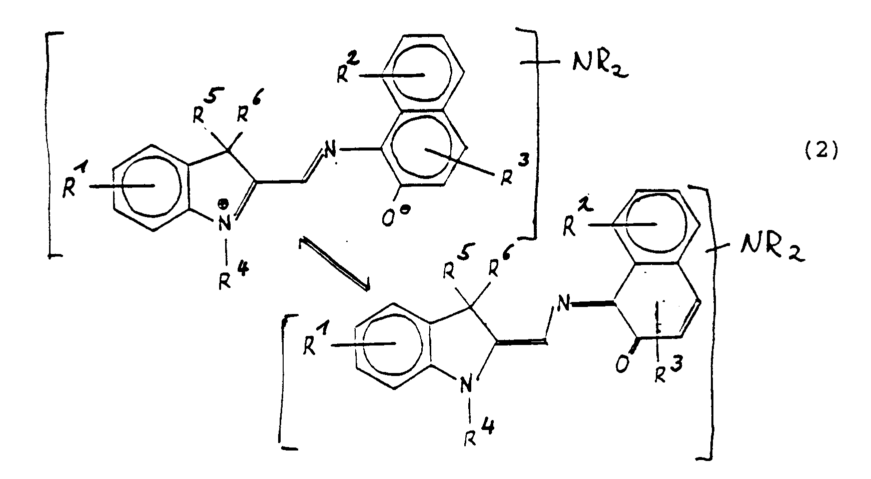
Die Moleküle mit der Strukturformel (2) haben eine blaue Farbe, während das Molekül mit der Strukturformel (1) farblos ist.

Durch steigende Temperaturen wird die Rückreaktion begünstigt, d.h. das Gleichgewicht zwischen Molekülen mit der Strukturformel (1) und (2) wird bei gleicher Beleuchtungsstärke durch steigende Temperatur von der Modifikation (2) zu der Grundform (1) hin verschoben.

Der erfindungsgemäß vorgesehene Rest -NR₂ stellt eine starke sterische und zusätzlich eine elektronische Hinderung für die Rückreaktion dar. Dies trifft in etwas geringerem Maße auch für die Reste R⁴, R⁵ und R⁶ zu.

Sind die Reste R⁵ und R⁶ "größer" als Methyl, so weisen die erfindungsgemäßen photochromen Substanzen den zusätzlichen Vorteil der positiven Thermochromie auf, d.h. die Eindunkelung nimmt mit steigender Temperatur zu. Nach einer ebenfalls von den Erfindern entwickelten Modellvorstellung öffnet sich bei Verbindungen mit positiver Thermochromie ebenfalls die Spiro-C-O-Bindung, und es dürften folgende Moleküle entstehen, die eine blau-violette Farbe haben: 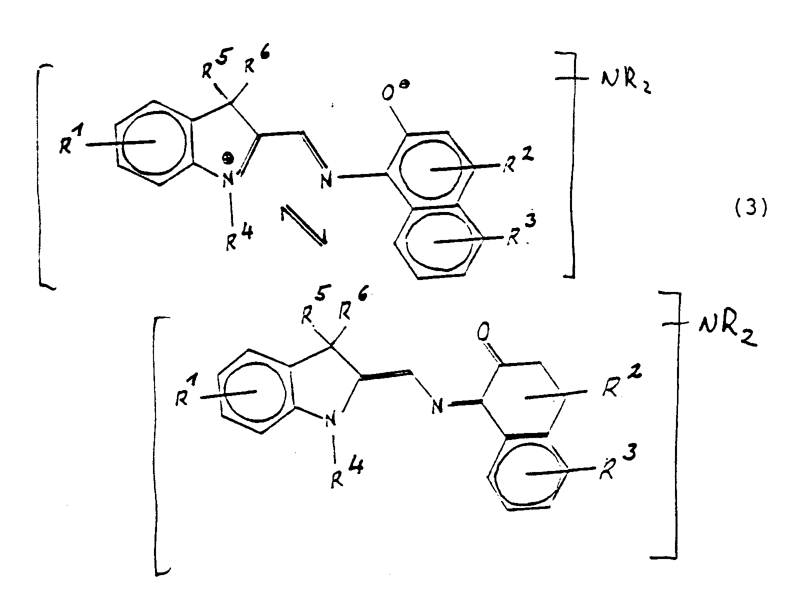
Das Gleichgewicht zwischen der Grundform (1) und der Modifikation (3) hängt nur von der Temperatur, nicht jedoch von der Beleuchtungsstärke ab.

Solche photo- und thermochromen Verbindungen sind in einer Matrix, beispielsweise einem optischen Element, ausschließlich phototropen Verbindungen, wie sie beispielsweise in der US-A-3 578 602 beschrieben sind, auch unter der Annahme gleicher phototroper Eigenschaften dadurch überlegen, daß sie im Sonnenlicht durch das durch dieses bewirkte Aufheizen der Matrix eine stärkere Eindunkelung aufweisen.

Die erfindungsgemäßen photochromen Substanzen haben den zusätzlichen Vorteil, daß bei gleicher Konzentration der Moleküle in einer Matrix, beispielsweise einem optischen Element, die Eindunkelung größer ist als bei den Substanzen, die in den eingangs genannten Durckschriften beschrieben sind.

Im Anspruch 2 sind besonders vorteilhafte Verbindungen für die Restgruppe -NR₂ angegeben. Bei Verwendung dieser Reste ist die beleuchtungsabhängige Einfärbung der photochromen Verbindung blau bis violett.

In den Ansprüchen 4 bis 6 sind Möglichkeiten gekennzeichnet, wie optische Elemente mit der erfindungsgemäßen photochromen Verbindung hergestellt werden können.

In besonders vorteilhafter Weise lassen sich die erfindungsgemäßen photochromen Substanzen bei einem Sonnenschutz-Brillenglas aus einem Kunststoffmaterial einsetzen.

Im folgenden soll ein Beispiel für eine erfindungsgemäße photochrome Verbindung vorgestellt werden:
17,1g (0,1mol) 1-Nitroso-2-napthol wird in 150 ml Trichlorethylen suspendiert, mit 17g (0,2 mol) Piperidin versetzt und unter Rückfluß bis zur vollständigen Lösung des Naphthols gekocht.

21,5g (0,1 mol)-1,1,3-Trimethyl-2-methylen-indolin werden in 100 ml Trichlorethylen gelöst und langsam zur ersteren Lösung bei Siedehitze zugetropft. Das Gemisch wird anschließend noch ca. 3 Stunden unter Rückfluß gekocht.

Die Lösung wird auf ca. 1/3 ihres Volumens eingeengt und über Al₂O₃ mit Trichlorethylen als Laufmittel chromatographiert. Aus dem ersten Eluat kann die nicht umgesetzte basische Ausgangsverbindung zurückgewonnen werden, die grüne Hauptzone enthält das Produkt. Das Laufmittel wird im Vakuum abgezogen.

Das gereinigte Piperidylderivat von 1,3,3-Trimethyl-spiro(indolino-2'--2,3'-(3H)naphth(2,1-b)(1.4)oxazin) besitzt einen Schmelzpunkt von 226°C. Die Verbindung hat dabei die folgenden 1H-NMR-Daten in ppm:

| | | |
|---|---|---|
| δ(-CH₃) | = 1,35 | (S) R⁵, R⁶ |
| δ(-CH₂-) | = 1,75 | (S) -NR₂ |
| δ(N-CH₃) | = 2,75 | (S) R⁴ |

Entsprechend diesen NMR-Daten hat die Verbindung die allgemeine Formel: 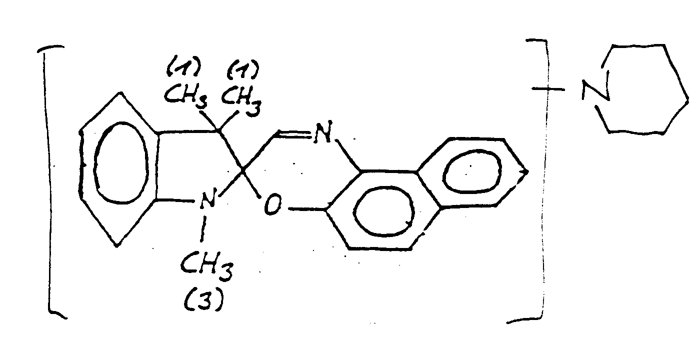
wobei der Rest -NR₂ an einer durch das Herstellverfahren vorgegebenen und durch die vorstehenden NMR-Daten näher spezifizierten Stelle mit dem Spirooxazin-Grundgerüst verbunden ist.

In folgenden soll exemplarisch eine Möglichkeit beschrieben werden, wie erfindungsgemäße Verbindungen, beispielsweise die Substanz, deren Herstellung vorstehend beschrieben worden ist, in eine Matrix eingebracht werden können.

Bei den folgenden Ausführungsbeispielen wird als Matrix eine 2mm dicke Planlinse aus Diethylenglykol-bis-allylcarbonat verwendet, das beispielsweise unter dem Warenzeichen CR 39 vertrieben wird.

Durch Einrühren von ca 5 Gew.% des Farbstoffs in ein Silikonöl oder in ein ähnliches hochsiedendes inertes Lösemittel wird ein Färbebad bereitet.

Die Kunststofflinse wird nach entsprechender Vorwärmung ca 20 min. lang in dieses Bad getaucht. Die Temperatur des Bads richtet sich nach dem Schmelzpunkt der photochromen Verbindung, sie liegt etwa 10°C über diesem. Da Temperaturen über 200°C die Matrix zerstören, werden höher schmelzende Substanzen bei 180°C in max. 30 min eingebracht. Nach dem Abkühlen wird die Linse mit Methylenchlorid gewaschen, der Farbstoff ist beidseitig in das Kunststoffmaterial eindiffundiert. Diese Einfärbung kann gegebenenfalls mehrfach wiederholt werden.

Die mit den erfindungsgemäßen photochromen Verbindungen eingefärbten Kunststofflinsen zeigen verglichen mit dem Stand der Technik ein wesentlich verbessertes Transmissionsverhalten:
Zur Messung der Temperaturabhängigkeit des photochromen Effekts wurden die Linsen nach dem vorstehend beschriebenen Verfahren so eingefärbt, daß der nach V_{λ}-bewertete Transmissionsunterschied zwischen unbelichtetem und belichtetem Glas bei der Standardtemperatur 23°C nach DIN 58217 gemessen annähernd gleich war. Die Messung erfolgte in einer Kinetikbank mit temperierbarer Küvette, die Belichtung erfolgte unter einem Winkel von 30° mit einer Beleuchtungsstärke von 60 000 lux. Das Spektrum der verwendeten Xenonhochdrucklampe war durch Filterkombinationen dem Spektrum des natürlichen Sonnenlichts auf Meereshöhe weitgehend angepaßt. Gemessen wurde die nach der spektralen Empfindlichkeit des menschlichen Auges bewertete Transmission nach 15-minütiger Bestrahlung. Zum Ausbleichen wurden die Gläser 30 min bei 90°C ausgeheizt und anschließend im Dunkeln auf Raumtemperatur abgekühlt.

**Tabelle**

| Unterschied in der Lichtdurchlässigkeit | | | |
|---|---|---|---|
| Temp. | Beispiel | Vergl.-subst.1 | Vergl. subst.2 |
| 10°C | 62,5 | 72,5 | 65,5 |
| 20°C | 59,5 | 61,5 | 52 |
| 30°C | 40,5 | 35,5 | 31 |
| Δ(Δτ) | 22 | 37 | 34 |

Die in der Tabelle aufgeführten Verbindungen haben folgende Reste in der allgemeinen Formel (1) (die Vergleichssustanzen sind der US-A-3 578 602 und der US-A-4 215 010 entnommen) :

| | | R^{1,3} | R² | R⁴ | R⁵ | R⁶ | -NR₂ |
|---|---|---|---|---|---|---|---|
| Beispiel | | H | H | CH₃ | CH₃ | CH₃ | NC₅H₁₀ |
| Vergleichss. | 1 | H | H | CH₃ | CH₃ | CH₃ | - |
| | 2 | H | 9-OCH₃ | CH₃ | CH₃ | CH₃ | - |

Wie aus der Tabelle ersichtlich ist, zeigen die erfindungsgemäßen Verbindungen bei gleicher oder sogar geringerer Eindunkelung als die bislang bekannten Verbindungen bei 10°C, einen stärkeren photochromen Effekt als diese bei 30°C. Der störende Abfall der Eindunkelung mit steigender Temperatur bei gleicher Beleuchtungsstärke, als Δ(Δτ) zwischen 10°C und 20°C und 30°C bewertet, wird um bis zu 40% reduziert.

## Patentansprüche

1. Photochrome Verbindung, erhalten in an sich bekannter Weise durch die Kondensation
- eines substituierten 2-Alkylenindolins 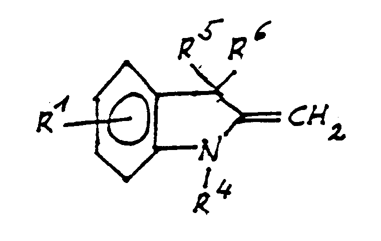
- mit einem 2-Hydroxy-1-nitroso-aromaten 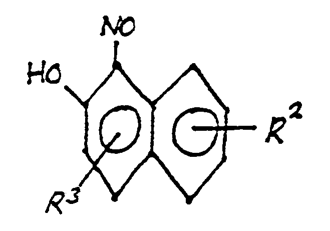
- in Anwesenheit eines Kondensationsmittels der Formel HNR₂, das den Rest -NR₂ in das Reaktionsprodukt einführt,
wobei die Reste
R¹,R²,R³ einen oder mehrere Substituenten aus der Reihe -H, -CN, -SCN, NO₂, -X, -CH₂X, -CX₃, -OR, -SR, -COR, -COOR darstellen,
wobei X = Halogen und R = H, Alkyl mit bis zu 8 C-Atomen, Aryl, Heteroaryl sind bzw. annelierte aromatische oder heteroaromatische Ringsysteme bedeuten,
R⁴,R⁵,R⁶ ein Substituent aus der Reihe -R⁷, -CH₂R⁷, ist
wobei R⁷ = Alkyl mit bis zu 8 C-Atomen oder Aryl bedeutet, und
R Alkyl mit bis zu 8 C-Atomen, Aryl oder Cyclohexyl ist,
oder die beiden R zusammen mit dem N-Atom einen Piperidylrest bilden.

2. Verbindungen nach Anspruch 1,
dadurch **gekennzeichnet**, daß der Rest -NR₂ die Bedentungen, -NC₅H₁₀ oder N(C₆H₁₁)₂ besitzt.

3. Optisches Element,
dadurch **gekennzeichnet,** daß es eine wirksame Menge einer photochromen Verbindung nach Anspruch 1 oder 2 enthält, die in das optische Element eingebracht oder als Schicht auf einem Kunststoffträgermaterial aufgebracht ist.

4. Optisches Element nach Anspruch 3,
dadurch **gekennzeichnet**, daß das optische Element aus einem Kunststoffmaterial besteht.

5. Optisches Element nach Anspruch 3 oder 4,
dadurch **gekennzeichnet**, daß das Element dadurch hergestellt ist,
daß die photochrome Verbindung in ein Silikonöl oder ein ähnliches hochsiedendes inertes Lösemittel eingerührt wird, daß die Mischung auf eine Temperatur etwa 10° oberhalb des Schmelzpunktes der photochromen Verbindung, maximal jedoch 180°C erwärmt wird, und daß das optische Grundelement maximal 30 min in die Mischung getaucht wird.

## Claims

1. A photochromic compound obtained in a manner known per se by condensation of
- a substituted 2-alkylene indoline 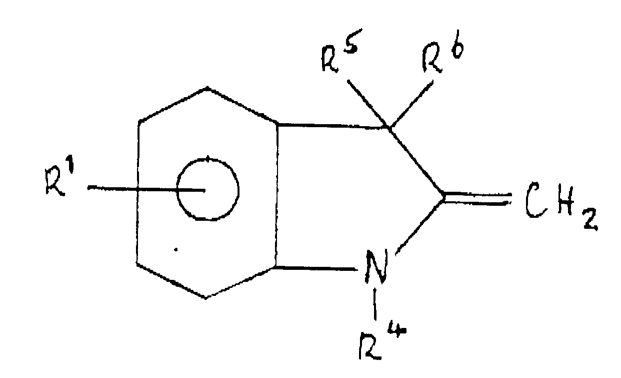
- with a 2-hydroxy-1-nitroso aromatic substance 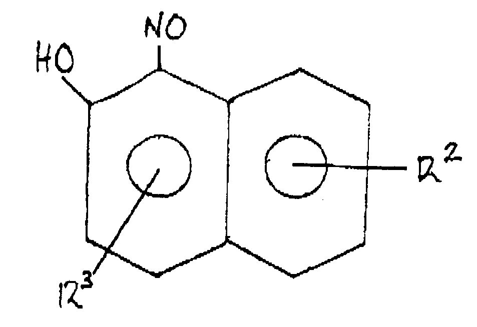
- in the presence of a condensation agent of the formula HNR₂ which introduces the residue -NR₂ into the reaction product, where the residues
R¹, R² R³ represent one or more substituents from the series -H, -CN, -SCN, -NO₂, -X, -CH₂X, -CX₃, -OR, -SR, -COR, -COOR, where X = halogen and R = H, or is alkyl with up to 8 C atoms, aryl, or heteroaryl or means fused aromatic or heteroaromatic ring systems,
R⁴, R⁵, R⁶ is a substituent from the series -R⁷, -CH₂R⁷,
where R⁷ = alkyl with up to 8 C atoms or aryl, and R is alkyl with up to 8 C atoms, aryl or cyclohexyl or the two R's form a piperidyl residue together with the N-atom.

2. Compounds according to claim 1, characterized in that the residue -NR₂ is -NC₅H₁₀ or N(C₆H₁₁)₂.

3. An optical element characterized in that it contains an effective amount of a photochromic compound according to claim 1 or 2, which is introduced into the optical element or applied as a layer to a plastics carrier material.

4. An optical element according to claim 3, characterized in that the optical element consists of a plastics material.

5. An optical element according to claim 3 or 4, characterized in that the element is manufactured by stirring the photochromic compound into a silicone oil or a similar inert solvent with high boiling point, by heating the mixture to a temperature around 10° above the melting point of the photochromic compound, to a maximum however of 180°C, and by dipping the optical base element into the mixture for a maximum of 30 minutes.

## Revendications

1. Composé photochromique, obtenu de façon connue en soi par la condensation :
- d'une 2-alkylène indoline substituée 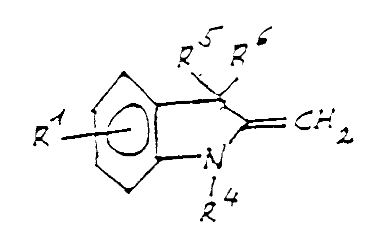
- avec un composé 2-hydroxy-1-nitroso-aromatique 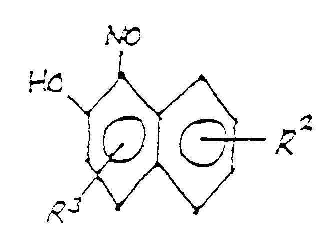
- en présence d'un agent de condensation de formule HNR₂, qui introduit le reste -NR₂ dans le produit de réaction,
les restes R¹, R², R³ représentant un ou plusieurs substituants choisis dans la série formée par -H, -CN, -SCN, -NO₂, -X, -CH₂X, -CX₃, -OR, -SR, -COR, -COOR,
X représentant un atome d'halogène et R représentant H, un groupe alkyle ayant jusqu'à 8 atomes de carbone, un groupe aryle ou hétéroaryle ou bien les restes R¹, R², R³ représentent des systèmes cycliques aromatiques ou hétéroaromatiques condensés,
les restes R⁴, R⁵, R⁶ représentent chacun un substituant choisi parmi -R⁷ et -CH₂R⁷, où R⁷ représente un groupe alkyle ayant jusqu'à 8 atomes de carbone ou un groupe aryle,
et R représente un groupe alkyle ayant jusqu'à 8 atomes de carbone, un groupe aryle ou cyclohexyle, ou bien les deux R forment, avec l'atome de N,, un reste pipéridyle.

2. Composés selon la revendication 1, caractérisés en ce que le reste -NR₂ possède les sens -NC₅H₁₀ ou N(C₆H₁₁)₂.

3. Elément optique, caractérisé en ce qu'il contient une quantité efficace d'un composé photochromique selon la revendication 1 ou 2, qui a été introduit dans l'élément optique ou a été appliqué sous forme de couche sur un matériau de support en matière plastique.

4. Elément optique selon la revendication 3, caractérisé en ce que l'élément optique consiste en une matière plastique.

5. Elément optique selon la revendication 3 ou 4, caractérisé en ce que l'élément est produit par incorporation par délayage du composé photochromique dans une huile de silicone ou dans un solvant inerte similaire à point élevé d'ébullition, en ce que le mélange est chauffé à une température supérieure d'environ 10° au point de fusion du composé photochromique mais au maximum à 180 °C, et en ce que l'élément optique de base est plongé durant 30 min au maximum dans le mélange.
